# EUROPÄISCHE PATENTANMELDUNG

(11) **EP 1 396 262 A1**
(43) Veröffentlichungstag der Anmeldung: **10.03.2004**
(21) Anmeldenummer: 03019963.2
(22) Anmeldetag: 03.09.2003
(51) Int. Cl.: A61K 7/48

(54) **Kosmetikum mit aktivierbaren Pflegekomponenten**

(30) Priorität: 05.09.2002 DE 10241073
(71) Anmelder: Beiersdorf AG, 20245 Hamburg (DE)
(72) Erfinder: Schepky, Andreas, Dr., 22459 Hamburg (DE); Ruppert, Stephan, Dr., 20259 Hamburg (DE); Counradi, Katrin, 22143 Hamburg (DE); Treu, Jens, Dr., 22844 Norderstedt (DE); Rohde, Olaf, 25335 Elmshorn (DE)

(57) **Zusammenfassung**

Kosmetikum und/oder Dermatikum enthaltend
a) eine kosmetische und/oder dermatologische Zubereitung A in einer Menge von 60 bis 99 Gewichts-% der Gesamtzubereitung, enthaltend ein oder mehrere kosmetische und/oder dermatologische Wirkstoffe, die in Form von stabilen Derivaten vorliegen,
b) eine Zubereitung B in einer Menge von 1 bis 40 Gewichts-% der Gesamtzubereitung, enthaltend ein oder mehrere Enzyme,
dadurch gekennzeichnet, dass die beiden Zubereitungen A und B in getrennten Kammern eines gemeinsamen Verpackungsbehältnisses aufbewahrt und aus diesem heraus angewendet werden, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

## Beschreibung

Die vorliegende Erfindung betrifft Kosmetika und/oder Dermatika aus zwei Komponenten.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut und der Hautanhangsgebilde.

Damit die Haut und die Hautanhangsgebilde, hierzu zählen vor allem die Haare und Nägel, ihre biologischen Funktionen im vollen Umfang erfüllen können, bedürfen sie der regelmäßigen Reinigung und Pflege sowie dem Schutz vor UV-Strahlung. Die Reinigung dient dabei der Entfernung von Schmutz, Schweiß und Resten abgestorbener Körperzellen, die einen idealen Nährboden für Krankheitserreger und Parasiten aller Art bilden. Kosmetische Reinigungsprodukte werden in der Regel in Form von Gelen, Lotionen und Feststoffen (Seifenstücke, Waschsynthets) angeboten. Hautpflegeprodukte, in der Regel Crémes, Salben oder Lotionen, dienen meist der Befeuchtung und Rückfettung der Haut, denn die Aufgabe der Hautpflege ist es, den durch das tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Häufig sind kosmetischen und dermatologischen Zubereitungen Wirkstoffe zugesetzt, welche die Haut regenerieren und beispielsweise ihre vorzeitige Alterung (z.B. das Entstehen von Fältchen, Falten) verhindern und vermindern sollen. Hierzu zählen unter anderem Vitamine, Coenzyme und Antioxidantien. Viele dieser Verbindungen sind hoch reaktiv, zersetzen sich leicht unter Licht- und/oder Sauerstoffeinfluss und lassen sich deshalb nicht stabil in kosmetische und/oder dermatologische Zubereitungen einarbeiten. So sind beispielsweise Vitamine meist nicht stabil in tensidhaltige Reinigungszubereitungen hinein zu formulieren. Aus diesem Grunde werden Wirkstoffe in der Regel nicht in ihrer physiologisch wirksamen Aktivform, sondern vielmehr in Form von chemisch stabileren Derivaten den kosmetischen und/oder dermatologsichen Zubereitungen zugesetzt. Die kosmetische und/oder dermatologische Wirkung derartiger Präparate beruht dann vermutlich darauf, dass die aktive Spezies der Wirkstoffe durch hauteigene Enzyme aus den Derivaten freigesetzt wird.

Nachteilig am Stande der Technik ist jedoch der Umstand, dass insbesondere bei Kosmetika und/oder Dermatika, die nur kurzzeitig in Kontakt zur Haut und Ihren Anhangsgebilden geraten (sogenannte rinse-off-Produkte), die Wirkung solcher Wirkstoffderivate vollkommen unzureichend ist. Dies ist vor allem bei Reinigungszubereitungen wie zum Beispiel Shampoos, Waschsynthets oder Duschgelen der Fall. Die kurze Einwirkungsdauer dieser Zubereitungen reicht in der Regel nicht aus, um die Wirkstoffe mit Hilfe der hauteigenen Enzyme aus ihren Derivaten freizusetzen.

Man hat versucht dieses Problem dadurch zu umgehen, dass man den kosmetischen Zubereitungen neben den Wirksstoffen in derivatisierter Form auch Enzymextrakte zusetzt, mit deren Hilfe die Wirkstoffe aus den Derivaten freigesetzt werden können. Doch werden bei diesen Zubereitungen die reaktiven Wirkstoffe auch schon vor der Anwendung auf der Haut und ihren Anhangsgebilden freigesetzt und anschließend durch Licht- und/oder Sauerstoffeinfluss zerstört, so dass die die Wirksamkeit dieser Produkte mit der Zeit deutlich abnimmt. Zum anderen wird die Wirksamkeit der eingesetzten Enzyme durch Bestandteile der Zubereitungen ganz oder teilweise zerstört, da es hier zu Unverträglichkeiten kommt. Dies ist insbesondere bei Tensiden der Fall, die den wesentlichen Bestandteil von Reinigungszubereitungen darstellen.

Es war daher die Aufgabe der vorliegenden Erfindung, die Mängel des Standes der Technik zu beseitigen oder zumindest zu lindern und Kosmetika bzw. Dermatika zu entwickeln, die bei der Anwendung hohe Konzentrationen an kosmetischen und/oder dermatologischen Wirkstoffen der Haut und ihren Anhangsgebilden zur Verfügung stellen und gleichzeitig über einen langen Zeitraum eine gleichbleibend hohe Wirkung aufweisen, d.h. langzeitstabil sind.

Überraschend gelöst wird die Aufgabe durch ein Kosmetikum und/oder Dermatikum enthaltend
a) eine kosmetische und/oder dermatologische Zubereitung A in einer Menge von 60 bis 99 Gewichts- % der Gesamtzubereitung, enthaltend ein oder mehrere kosmetische und/oder dermatologische Wirkstoffe, die in Form von stabilen Derivaten vorliegen,
b) eine Zubereitung B in einer Menge von 1 bis 40 Gewichts- % der Gesamtzubereitung, enthaltend ein oder mehrere Enzyme,
dadurch gekennzeichnet, dass die beiden Zubereitungen A und B in getrennten Kammern eines gemeinsamen Verpackungsbehältnisses aufbewahrt und aus diesem heraus angewendet werden, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

Mit Hilfe der erfindungsgemäßen Zubereitungen lassen sich hoch wirksame Kosmetika beziehungsweise Dermatika herstellen, die innerhalb kurzer Zeit große Mengen an freien Wirkstoffen der Haut und Ihren Anhangsgebilden zur Verfügung stellen und gleichzeitig eine hohe Transport-, Lager- und Temperaturstabilität aufweisen.

Besonders bevorzugt lassen sich mit Hilfe der erfindungsgemäßen Zubereitungen Reinigungspräparate darstellen, da diese meist im angewärmten Zustand (z.B. durch den Anwendung von Warmwasser) auf die Haut und ihre Anhangsgebilde appliziert werden. Da die Aktivität der Enzyme mit steigender Temperatur stark zunimmt, werden sehr schnell große Mengen an Wirkstoffen in ihrer physiologisch aktiven Form freigesetzt. Die vorgewärmte Haut und Ihre Anhangsgebilde sind im angewärmten Zustand durch die geöffneten Poren besonders aufnahmefähig für die freigesetzten Wirkstoffe, so dass sich die angebotene Menge an Wirkstoffen und Aufnahmefähigkeit durch die Haut und ihre Anhangsgebilde in idealer Weise ergänzen.

Es ist erfindungsgemäß vorteilhaft, wenn in der Zubereitung A als Wirkstoffe, die in derivatisierter Form vorliegen, Vitamine eingesetzt werden. Erfindungsgemäß bevorzugt werden dabei Retinylpalmitat, Ascorbylglucosid, Tocopherylacetat, Tocopherylpalmitat eingesetzt. Erfindungsgemäß besonders bevorzugt ist der Einsatz Retinylpalmitat.

Erfindungsgemäß vorteilhaft werden ein oder mehrere kosmetische und/oder dermatologische Wirkstoffe, die in Form von stabilen Derivaten vorliegen, in einer Gesamtkonzentration von 0,01 bis 10 Gewichts- %, bevorzugt in einer Konzentration 0,01 von bis 5 Gewichts- % und ganz besonders bevorzugt in einer Konzentration von 0,05 bis 1 Gewichts- %, jeweils bezogen auf das Gesamtgewicht der Zubereitung A, dieser Zubereitung zugefügt.

Erfindungsgemäß vorteilhaft ist der Einsatz von Retinylpalmitat in einer Konzentration von 0,05 bis 1 Gewichts- %, bevorzugt in einer Konzentration von 0,01 bis 0,5 Gewichts-% und ganz besonders bevorzugt in einer Konzentration von 0,05 bis 0,2 Gewichts-%, jeweils bezogen auf das Gesamtgewicht der Zubereitung A.

Das erfindungsgemäße Kosmetikum und/oder Dermatikum enthält vorteilhaft in Zubereitung B als Enzyme Lipasen, Esterasen, Proteasen und andere Hydrolasen. Dabei sind Lipasen erfindungsgemäß bevorzugt.

Als erfindungsgemäße Enzyme können vorteilhaft Enzymextrakte aus *Alcaligenes sp.* (z.B. von der Firma Amano Enzyme Europe Ltd.), *Aspergillus niger* (z.B. von den Firmen Amano Enzyme Europe Ltd. oder Fluka), *Candida cylindracea* (z.B. von den Firmen Sigma, Aldrich, Fluka, Amano Enzyme Europe Ltd., Boeringer-Mannheim), *Candida* lipolytica (z.B. von den Firmen Amano Enzyme Europe Ltd., Fluka), *Chromobacterium viscosium* (z.B. von den Firmen Sigma), *Humicola laguninosa* (z.B. von der Firma Amano Enzyme Europe Ltd.), *Mucor miehei* (z.B. von den Firmen Amano Enzyme Europe Ltd., Novo), *Penecillium* roqueforti (z.B. von der Firma Fluka), *Porcine pancreas* (z.B. von den Firmen Sigma, Aldrich, Fluka, Amano Enzyme Europe Ltd., Boeringer-Mannheim), *Pseudomonas aegruginosa* (z.B. von der Firma Amano Enzyme Europe Ltd.), *Pseudomonas* fluorescens (z.B. von den Firmen Amano Enzyme Europe Ltd., Fluka), *Pseudomonas sp.*(z.B. von den Firmen Sigma, Boeringer-Mannheim), *Rhizopus delemar* (z.B. von der Firma Sigma, Fluka, Boeringer-Mannheim), *Rhizopus japonicus* (z.B. von den Firmen Amano Enzyme Europe Ltd.), *Rhizopus oryzae* (z.B. von der Firma Amano Enzyme Europe Ltd.), *Rhizopus sp.* (z.B. von den Firmen Amano Enzyme Europe Ltd., Serva) und/oder Weizenkeim (z.B.von der Firma Sigma) eingesetzt werden.

Dabei sind als Enzym-Extrakte Lipasen aus Schweinepankreas (Porcine pancreas), *Aspergillus niger, Chromobacterium viscosium, Geotrichum candidum, Penecillium camberti, Penecillium roqueforti* erfindungsgemäß bevorzugt.

Erfindungsgemäß ganz besonders bevorzugt sind Lipasen der Art *Mucor miehei* (z.B. Lipozyme® von der Firma Novo Nordisk ), *Humicola laguninosa, Pseudomonas aegruginosa, Pseudomonas fluorescens, Pseudomonas sp., Candida cylindracea* und/oder *Candida lipolytica.*

Erfindungsgemäß vorteilhaft liegt die Zubereitung B in Form einer wässrigen Lösung vor. Deren Gehalt an einem oder mehreren Enzymen beträgt erfindungsgemäß vorteilhaft von 0,1 bis 5 Gewichts- %, bevorzugt von 1 bis 5 Gewichts- % und ganz besonders bevorzugt von 1 bis 2 Gewichts-%, jeweils bezogen auf das Gesamtgewicht der Zubereitung B.

Erfindungsgemäß vorteilhaft beträgt die Konzentration an Lipasen aus *Humicola laguninosa* von 0,1 bis 5 Gewichts- %, bevorzugt von 1 bis 5 Gewichts- % und ganz besonders bevorzugt von 1 bis 2 Gewichts- %, jeweils bezogen auf das Gesamtgewicht der Zubereitung B.

Erfindungsgemäß vorteilhaft beträgt die Konzentration an Lipasen aus *Mucor miehei* von bis Gewichts-%, bevorzugt von 0,1 bis 5 Gewichts- %, bevorzugt von 1 bis 5 Gewichts- % und ganz besonders bevorzugt von 1 bis 2 Gewichts- %, jeweils bezogen auf das Gesamtgewicht der Zubereitung B.

Erfindungsgemäß vorteilhaft beträgt die Konzentration an Lipasen aus *Pseudomonas aegruginosa* von 0,1 bis 5 Gewichts- %, bevorzugt von 1 bis 5 Gewichts- % und ganz besonders bevorzugt von 1 bis 2 Gewichts- %, jeweils bezogen auf das Gesamtgewicht der Zubereitung B.

Es ist erfindungsgemäß vorteilhaft, wenn als Zubereitung A eine tensidhaltige Reinigungszubereitung eingesetzt wird.

Die erfindungsgemäße Zubereitung A kann vorteilhafter Weise ein oder mehrere Tenside enthalten. Erfindungsgemäß vorteilhaft können sowohl anionische, kationische, nichtionische und zwitterionische Tenside eingesetzt werden. Erfindungsgemäß bevorzugt ist insbesondere der Einsatz von einem oder mehreren anionischen Tensiden in der Zubereitung A.

Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole,
■ ethoxylierte Amine
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders vorteilhaft, wenn als Tenside in der Zubereitung A Natriumlaurethsulfat, Natriummyrethsulfat, Cocamidopropylbetain, Laurylglucosid, Natriumcocoylglutamat und/oder Natriumcocoamphoacetat eingesetzt werden.

Erfindungsgemäß bevorzugt sind Tensidkombinationen aus Alkylethersulfaten mit amphoteren Cotensiden, wobei Tensidkombinationen aus Natriumlaurethsulfat und Cocamidopropylbetain oder Natriumlaurethsulfat, Cocamidopropylbetain und Natriumcocoylglutamat einerseits sowie Tensidkombinationen aus Natriummyrethsulfat und Decylglycosid andererseits besonders bevorzugt ist. Insbesondere mit diesen Tensidkombinationen lassen sich Reinigungszubereitungen mit hoher Schaumleistung, sowie besonders cremigem Schaum bei der Anwendung herstellen.
Es ist vorteilhaft im Sinn der vorliegenden Erfindung, wenn der Gehalt an einem oder mehreren waschaktiven Tensiden in der kosmetischen Zubereitung A aus dem Bereich von 1 bis 30 Gewichts-%, bevorzugt von 5 bis 25 Gew.-%, ganz besonders vorteilhaft von 10 bis 20 Gew.-% gewählt wird, jeweils bezogen auf das Gesamtgewicht der Zubereitung A.

Erfindungsgemäß vorteilhaft kann die Zubereitung A Polysorbate enthalten.

Im Sinne der Erfindung vorteilhafte Polysorbate sind beispielsweise das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Pölyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).

Diese werden erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der Zubereitung A einzeln oder als Mischung mehrer Polysorbate, eingesetzt.

Die erfindungsgemäßen Zubereitungen A und B können als wässrige Lösung oder wässrige Phase einer Emulsion neben Wasser erfindungsgemäß auch andere Inhaltsstoffe enthalten, beispielsweise Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol und Glycerin.

Die kosmetischen Zubereitungen A und B können zusätzlich neben einer oder mehreren Wasserphasen eine oder mehrere Ölphasen enthalten und beispielsweise in Form von W/O-, O/W-, W/O/W- oder O/W/O-Emulsionen vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion) sein, wobei transparente oder transluzente Mikroemulsionen erfindungsgemäß besonders bevorzugt sind.

Die erfindungsgemäßen Zubereitungen können vorteilhaft anfeuchtende bzw. feuchthaltende Mittel (sogenannte Moisturizer) enthalten. Vorteilhafte feuchthaltende Mittel im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der jeweiligen Zubereitung.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin und deren Derivate.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

In die erfindungsgemäßen Zubereitungen können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin, Antihistaminika, Antiphlogistika, Glucocorticoide (z.B. Hydrocortison) sowie Pflanzenwirkstoffe wie Azulen und Bisabolol, Glycyrrhizin, Hamamelin und Pflanzenextrakte wie Kamille, aloe vera, Hamazelis, Süßholzwurzel. Auch die Vitamin D₃-analoga Tacalcitol, Calcipotriol, Tacalcitol, Colecalciferol sowie Calcitrol (Vitamin D₃) und/oder Fumarsäureester können erfolgreich in die Zubereitungen hineinformuliert werden.

Die Ölphase bzw. Ölphasen der erfindungsgemäßen Formulierungen wird/werden vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann eine Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Cocoglyceride (z. B. Myritol® 331 von Henkel), C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Corapan*®*TQ von Haarmann & Reimer*).

Auch beliebige Abmischungen solcher Öl- und Wachskomponenten sind vorteilhaft im Sinne der vorliegenden Erfindung einzusetzen.

Ferner kann eine Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann eine Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß bevorzugt sind Kosmetika bzw. Dermatika, deren Zubereitung A eine wässrige Lösung oder ein Gel darstellen.

Erfindungsgemäß bevorzugt sind Kosmetika bzw. Dermatika, deren Zubereitung B eine wässrige Lösung oder ein Gel darstellen.

Die erfindungsgemäßen Zubereitungen können auch alle nach der Kosmetikverordnung zugelassenen wasserlöslichen und/oder öllöslichen UV-A-, UV-B- und/oder Breitbandfiltersubstanzen enthalten.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, weitere Pflanzenextrakte, Vitamine, Wirkstoffe, Konservierungsmittel, Bakterizide, Repellentien, Selbstbräuner, Depigmentierungsmittel, Pigmente, die eine färbende Wirkung haben, weichmachende, anfeuchtende und/oder feuchthaltende Substanzen, oder andere übliche Bestandteile einer kosmetischen oder dermatologischen Formulierung wie Emulgatoren, Polymere, Schaumstabilisatoren und Elektrolyte.

Die erfindungsgemäßen Zubereitungen A und B können erfindungsgemäß vorteilhaft ein oder mehrere Konservierungsstoffe enthalten. Vorteilhafte Konservierungsstoffe im Sinne der vorliegenden Erfindung sind beispielsweise Formaldehydabspalter (wie z. B. DMDM Hydantoin, welches beispielsweise unter der Handelsbezeichnung Glydant ^{TM} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfasst das Konservierungssystem erfindungsgemäß ferner vorteilhaft auch Konservierungshelfer, wie beispielsweise Octoxyglycerin, Glycine Soja etc. Die nachfolgende Tabelle gibt einen Überblick über einige erfindungsgemäß vorteilhafte Konservierungsstoffe:

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propylester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisulfit | E 282 | Calciumpropionat |
| E 224 | Kaliumdisulfit | E 283 | Kaliumpropionat |
| E 226 | Calciumsulfit | E 290 | Kohlendioxid |

Ferner vorteilhaft sind in der Kosmetik gebräuchliche Konservierungsmittel oder Konservierungshilfsstoffe, wie Dibromdicyanobutan (2-Brom-2-brommethylglutarodinitril), Phenoxyethanol, 3-lod-2-propinylbutylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol.
Es ist dabei erfindungsgemäß besonders bevorzugt, wenn als Konservierungsstoffe Benzoesäure und/oder Salicylsäure und/oder deren Derivate oder Salze eingesetzt werden.

Erfindungsgemäß vorteilhaft sind ein oder mehrere Konservierungsstoffe in einer Konzentration von 2 Gewichts-% oder kleiner 2 Gewichts-%, bevorzugt 1,5 Gewichts-% oder kleiner 1,5 Gewichts-% und besonders bevorzugt 1 Gewichts-% oder kleiner 1 Gewichts-%, jeweils bezogen auf das Gesamtgewicht der Zubereitung A oder B in einer der beiden Teilzubereitungen oder in beiden Teilzubereitungen enthalten. Erfindungsgemäß bevorzugt befinden sich ein oder mehrere Konservierungsmittel in der Zubereitung A.

Die erfindungsgemäßen Zubereitungen enthalten vorteilhafter Weise einen oder mehrere Konditionierer. Erfindungsgemäß bevorzugte Konditionierer sind beispielsweise alle Verbindungen, welche im *International Cosmetic Ingredient Dictionary and Handbook* (Volume 4, Herausgeber: R. C. Pepe, J.A. ,Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Ein Teil dieser Verbindungen wird unter den Bestandteilen der wässrigen Phase und der Ölphase namentlich aufgeführt. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Es ist erfindungsgemäß vorteilhaft, den mindestens einer der erfindungsgemäßen Zubereitungen Glitterstoffe und/oder andere Effektstoffe zuzusetzen.

Auch ist es vorteilhaft im Sinne der vorliegenden Erfindung, wenn mindestens eine der beiden Zubereitungen A und B ein oder mehrere Hydrokolloide in einer Konzentration von 0,01 bis 10 Gewichts-%, bevorzugt in einer Konzentration von 0,1 bis 7 Gewichts-% und ganz besonders bevorzugt in einer Konzentration von 0,3 bis 5 Gewichts-%, jeweils bezogen auf das Gesamtgewicht der Zubereitung enthält.

Als erfindungsgemäß vorteilhafte Hydrokolloide werden Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Celluloseether, Hydroxyethyl- und -propylcellulosederivate, Polysaccharide, Polyacryl- und Polymethacryl-Verbindungen, Vinylpolymere, Polycarbonsäuren, Polyether, Polyimine, Polyamide, Polykieselsäuren, Tonmineralien, Zeolithe, Kieselsäuren eingesetzt.

Erfindungsgemäß bevorzugte Hydrokolloide sind beispielsweise Methylcellulosen, als welche die Methylether der Cellulose bezeichnet werden.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Spezies unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Spezies unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet: α, β, γ, µ, v, ξ, π, ω, χ. Auch die Art vorhandener Kationen (K+, NH4+, Na+, Mg2+, Ca2+) beeinflusst die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

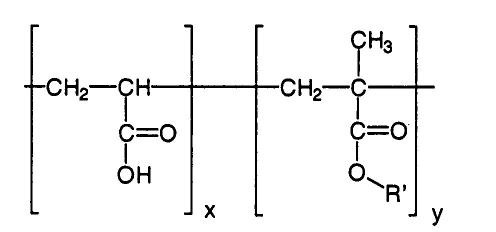

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Erfindungsgemäß bevorzugt ist es insbesondere, neutralisierte oder teilneutralisierte Polyacrylate (z.B. Carbopole der Firma Noveon) einzusetzen.

Die Gesamtmenge an einem oder mehreren Hydrokolloiden wird vorteilhaft kleiner als 5 Gew.-%, bevorzugt zwischen 0,1 und 5 Gew.-%, bezogen auf das Gesamtgewicht der jeweiligen Zubereitung, gewählt.

Es ist vorteilhaft im Sinn der vorliegenden Erfindung, wenn der Gehalt an einem oder mehreren Polyacrylaten in einer kosmetischen oder dermatologischen Reinigungsemulsion aus dem Bereich von 0,1 bis 8 Gew.-%, ganz besonders vorteilhaft von 0,1 bis 5 Gew.-% gewählt wird, jeweils bezogen auf das Gesamtgewicht der jeweiligen Zubereitung.

Erfindungsgemäße Verpackungsbehältnisse können vorteilhaft aus einer Kunststoffflasche oder einer Aerosoldose bestehen. Erfindungsgemäß vorteilhaft kann auch mindestens eine der beiden Zubereitungen mit einem Treibmittel aufgeschäumt werden.

Als erfindungsgemäß vorteilhafte Verpackungsbehältnisse können beispielsweise Verpackungen gewählt werden, wie sie in der WO 96/37420 oder der GB 2 180 215 offenbart sind. Auch Verpackungssysteme wie sie von mehrfarbig gestreifter Zahnpasta her bekannt sind, lassen sich erfindungsgemäß vorteilhaft verwenden unter der Maßgabe, dass die beiden Zubereitungen durch eine undurchlässige Sperre voneinander getrennt sind und erst beim Austritt aus der Verpackung zu der bekannten streifenförmigen Gesamtzusammensetzung zusammengesetzt werden.

Vorteilhafte Verpackungsbehältnisse stellen ferner mit zwei Kammern versehene Quetschflaschen dar, die entweder für beide Zubereitungen eine gemeinsame Entnahmeöffnung oder aber zwei separate Entnahmeöffnungen aufweisen.

Erfindungsgemäß vorteilhafte Verpackungsbehältnisse stellen auch Doppelkammertuben mit ein oder zwei Entnahmeöffnungen dar.

Nicht zuletzt ist es erfindungsgemäß vorteilhaft, wenn beide Zubereitungen zusammen oder getrennt mit Hilfe eines Pump-und/oder Dosierspenders dem Verpackungsbehältnis entnommen werden können.

Generell bevorzugt sind Verpackungsbehältnisse, bei denen die beiden erfindungsgemäßen Zubereitungen A und B dem Behältnis aus zwei unterschiedlichen Entnahmeöffnungen entnommen werden.

Es ist erfindungsgemäß vorteilhaft, wenn die beiden Zubereitungen A und B unterschiedlich gefärbt und/oder mit unterschiedlichen Effektstoffen beladen sind.

Erfindungsgemäß ist die Verwendung des erfindungsgemäßen Kosmetikums und/oder Dermatikums zur Reinigung und Pflege der Haut und/oder Hautanhangsgebilde.

Insbesondere ist die Verwendung des erfindungsgemäßen Kosmetikums und/oder Dermatikums als Duschgel, Wannenbad oder Haarwaschmittel (Haarshampoo) erfindungsgemäß.

Doch ist auch die Verwendung der erfindungsgemäßen Kosmetika und/oder Dermatika als Hautcreme, Lotion oder Gel zur Pflege der Haut erfindungsgemäß vorteilhaft.

Ferner eignen sich die erfindungsgemäßen Produkte hervorragend zur Reinigung und Pflege von Gegenständen des täglichen Lebens (z.B. Geschirr, Tisch- und Schrankflächen, Autos).

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

| **Phase1** | | | | | | |
|---|---|---|---|---|---|---|
| | **1** | **2** | **3** | **4** | **5** | **6** |
| Natrium Laurethsulfat | 13,2% | 11 % | 10% | 11 % | 12% | 12% |
| Cocoamidopropylbetain | 1,65% | 3,3% | 1,5% | 3,3% | 3% | 3% |
| Natriumcocoylglutamat | 1,25% | 0,75% | --- | 0,75% | --- | --- |
| Acrylat Copolymer | 0,5% | --- | --- | 0,7% | --- | --- |
| Alkylpolyglucosid | --- | --- | 3% | --- | 3% | 3% |
| PEG-40 hydriertes Rizinusöl | 0,50% | 0,50% | 0,50% | 0,50% | 0,50% | 0,50% |
| PEG-100 hydriertes Glycerylpalmitat | 0,50% | 0,50% | 0,50% | 0,50% | 0,50% | 0,50% |
| Natriumbenzoat | 0,45% | 0,45% | 0,45% | 0,45% | 0,45% | 0,45% |
| Natriumsalicylat | 0,20% | 0,20% | 0,20% | 0,20% | 0,20% | 0,20% |
| Retinylpalmitat | 0,1% | - | - | - | 0,12% | - |
| Tocopherolacetat | - | 0,05% | - | - | 0,1% | - |
| Vitamin C Glucosid | - | - | 0,12% | - | 0,08% | 0,05% |
| Calziferolpropionat | - | - | - | 0,75% | - | 0,12% |
| Citronensäure | 0,50% | 0,50% | 0,50% | 0,50% | 0,50% | 0,50% |
| Parfum | q.s. | q.s. | q.s. | q.s. | q.s. | q.s. |
| Wasser | ad 100 | ad 100 | ad 100 | ad 100 | Ad 100 | ad 100 |

| **Phase2** | | | | | |
|---|---|---|---|---|---|
| | **1** | **2** | **3** | **4** | **5** |
| PEG - 20 | 0,1% | 0,2 % | 0,5% | 1% | 5% |
| Glycerin | 0,2% | 0,5% | 1 % | 2% | 5% |
| Lipase / Esterase | 1% | 2% | 3% | 4% | 5% |
| CaCl₂ | 0,1% | 0,1% | 0,1% | 0,1% | 0,1% |
| Propylparaben | 0,1% | 0,1 % | 0,1 % | 0,1 % | 0,5% |
| Wasser | ad 100 | ad 100 | ad 100 | ad 100 | ad 100 |

## Patentansprüche

1. Kosmetikum und/oder Dermatikum enthaltend
a) eine kosmetische und/oder dermatologische Zubereitung A in einer Menge von 60 bis 99 Gewichts-% der Gesamtzubereitung, enthaltend ein oder mehrere kosmetische und/oder dermatologische Wirkstoffe, die in Form von stabilen Derivaten vorliegen,
b) eine Zubereitung B in einer Menge von 1 bis 40 Gewichts-% der Gesamtzubereitung, enthaltend ein oder mehrere Enzyme,
**dadurch gekennzeichnet, dass** die beiden Zubereitungen A und B in getrennten Kammern eines gemeinsamen Verpackungsbehältnisses aufbewahrt und aus diesem heraus angewendet werden, wobei beide Zubereitungen dem Verpackungsbehältnis entweder durch eine gemeinsame Öffnung gleichzeitig entnommen werden und beide Zubereitungen entweder vor dem Austritt aus der Entnahmeöffnung vermischt oder in Form einer streifenförmigen Gesamtzubereitung aus der Entnahmeöffnung austreten, oder zwei getrennten Öffnungen entnommen werden.

2. Kosmetikum und/oder Dermatikum nach Anspruch 1, **dadurch gekennzeichnet, dass** in Zubereitung A als Wirkstoffe, die in derivatisierter Form vorliegen, Vitamine eingesetzt werden.

3. Kosmetikum und/oder Dermatikum nach einem der Ansprüche 1 oder 2, **dadurch gekennzeichnet, dass** in Zubereitung A als Wirkstoffe, die in derivatisierter Form vorliegen, Retinylpalmitat, Acsorbylglucosid, Tocopherylacetat, Tocopherylpalmitat eingesetzt werden.

4. Kosmetikum und/oder Dermatikum nach einem der Ansprüche 1 bis 3, **dadurch gekennzeichnet, dass** in Zubereitung B als Enzyme Lipasen, Esterasen, Proteasen und andere Hydrolasen eingesetzt werden.

5. Kosmetikum und/oder Dermatikum nach einem der Ansprüche 1 bis 4, **dadurch gekennzeichnet, dass** Zubereitung B eine wässrige Lösung darstellt, deren Gehalt an einem oder mehreren Enzymen von 1 bis 20 Gewichts-%, bezogen auf das Gesamtgewicht der Zubereitung B beträgt.

6. Kosmetikum und/oder Dermatikum nach einem der Ansprüche 1 bis 5, **dadurch gekennzeichnet, dass** in Zubereitung B als Enzyme Enzymextrakte aus *Alcaligenes sp., Achromobacter sp., Aspergillus niger, Bacillus subtilis, Candida cylindracea, Candida lipolytica, Chromobacterium viscosium, Geotrichum candidum, Humicola laguninosa, Mucor miehei, Penecillium camberti, Penecillium roqueforti, Porcine pancreas, Pseudomonas aegruginosa, Pseudomonas fluorescens, Pseudomonas sp.,* *Rhizopus delemar, Rhizopus japonicus, Rhizopus oryzae, Rhizopus sp.* und/oder Weizenkeim eingesetzt werden.

7. Kosmetikum und/oder Dermatikum nach einem der Ansprüche 1 bis 6, **dadurch gekennzeichnet, dass** als Zubereitung A eine tensidhaltige Reinigungszubereitung eingesetzt wird.

8. Kosmetikum und/oder Dermatikum nach einem der Ansprüche 1 bis 7, **dadurch gekennzeichnet, dass** die Zubereitung A ein oder mehrere kosmetische und/oder dermatologische Wirkstoffe, die in Form von stabilen Derivaten vorliegen, in einer Gesamtkonzentration von 1 bis 30 Gewichts-%, bezogen auf das Gesamtgewicht der Zubereitung A, vorliegen.

9. Verwendung eines Kosmetikums und/oder Dermatikums nach einem der vorhergehenden Ansprüche zur Reinigung und Pflege der Haut und/oder Hautanhangsgebilde.

10. Verwendung eines Kosmetikums und/oder Dermatikums nach einem der vorhergehenden Ansprüche als Duschgel, Wannenbad oder Haarwaschmittel.
